# EUROPEAN PATENT APPLICATION

(11) **EP 1 714 648 A1**
(43) Date of publication of application: **25.10.2006**
(21) Application number: 05709853.5
(22) Date of filing: 08.02.2005
(51) Int. Cl.: A61K 31/192, A61K 31/194, A61K 31/216, A61K 31/22, A61K 31/366, A61K 31/40, A61K 31/405

(54) **COMBINATION DRUG**

(30) Priority: 09.02.2004 JP 2004032329
(71) Applicant: ASKA Pharmaceutical Co., Ltd., Minato-ku, Tokyo 108-8532 (JP)
(72) Inventor: KANAZAWA, Hashime; Aska Pharma. Co., Ltd., Hamura-shi, Tokyo 2058501 (JP); ISHITANI, Kouki; Aska Pharma. Co., Ltd., Hamura-shi, Tokyo 2058501 (JP); SUDO, Katsuichi; Aska Pharma. Co., Ltd., Hamura-shi, Tokyo 2058501 (JP); TANIMORI, Naoto; Aska Pharma. Co., Ltd., Hamura-shi, Tokyo 2058501 (JP)
(74) Representative: von Kreisler, Alek
(86) International application number: PCT/JP2005/001801
(87) International publication number: WO 2005/074909

(57) **Abstract**

At least one hyperlipidemic agent selected from the group consisting of a fibrate compound (e.g., fenofibrate, bezafibrate, or a salt thereof) and an HMG-CoA reductase inhibitor (e.g. , a statin compound, for example, pravastatin, atorvastatin, or salts thereof) is, as active ingredients, combined with an a-glucosidase inhibitor (e.g. , voglibose, and acarbose) in the pharmaceutical composition of the present invention. The proportion of the α-glucosidase inhibitor may be about 0.001 to 50 parts by weight relative to 100 parts by weight of the hyperlipidemic agent. The present invention provides a pharmaceutical composition which is excellent in prophylactic and/or therapeutic effect on metabolic syndrome, hyperlipemia, diabetes, diabetes complications, etc, and has few side effects.

## Description

### TECHNICAL FIELD

The present invention relates to a pharmaceutical composition (a combined pharmaceutical composition, or combined medicament) useful as an agent for the prophylaxis and/or treatment of (an agent for preventing and/or treating) metabolic syndrome, hyperlipemia, diabetes, diabetes complications and other symptoms (or diseases); which includes (has) a combination of at least one hyperlipidemic agent (the agent for hyperlipemia) selected from the group consisting of a fibrate compound and a hydroxymethylglutaryl-CoA reductase inhibitor (e.g., a statin compound) with an α-glucosidase inhibitor.

### BACKGROUND ART

A fibrate compound is an agent having an action reducing a low density lipoprotein-binding cholesterol and a triglyceride by inhibiting synthesis or secretion of the triglyceride in liver, as well as increasing a high density lipoprotein-binding cholesterol, and has been widely known as an agent for the prophylaxis or treatment of hyperlipemia.

A statin compound is an agent having an action suppressing synthesis of a cholesterol by inhibiting hydroxymethylglutaryl-CoA (HMG-CoA) reductase which is a rate-determining enzyme in biosynthetic pathway of the cholesterol. The statin compound has been widely known as an agent for the prophylaxis or treatment of hyperlipemia as well as the fibrate compound.

There have been known to use either the fibrate compound or the statin compound alone, in addition, to use the fibrate compound or the statin compound in combination with a variety of drugs. For example, there have been known (1) a pharmaceutical composition containing a combination of fenofibrate and bezafibrate with metformin known as a therapeutic agent for diabetes, intended to reduce the hyperglycemia due to non-insulin-dependent diabetes(e.g., see Patent Document 1), (2) a therapeutic agent for hyperlipemia, atherosclerosis, or hypercholesterolemia, which comprises fenofibrate, bezafibrate, or clinofibrate, in combination with a cholesteryl ester transfer protein inhibiting compound (e.g., see Patent Document 2), (3) a therapeutic agent for hyperlipemia, atherosclerosis, or hypercholesterolemia, which comprises fenofibrate, bezafibrate, or clinofibrate, in combination with an ileal bile acid transport inhibiting compound (e.g., see Patent Document 3), (4) an agent for the prophylaxis or treatment of atherosclerosis, hypercholesterolemia, and hyperlipoproteinemia, which comprises lovastatin or cerivastatin, in combination with a β-blocker (e.g., see Patent Document 4), (5) a TNF-α-inhibitor useful as an agent for the prophylaxis or treatment of inflammatory disease, which comprises pravastatin or cerivastatin, in combination with pioglitazone known as an insulin sensitizer (e.g., see Patent Document 5), and (6) a therapeutic agent for angina pectoris, atherosclerosis, or a complication of hypertension and hyperlipidemia, which comprises pravastatin or simvastatin, in combination with amlodipine as a therapeutic agent for hypertension (e.g. , see Patent Document 6).

On the other hand, an α-glucosidase inhibitor is a drug having an action inhibiting a digestive enzyme such as an amylase, a maltase, an α-dexitrinase, or a sucrase and retarding digestion and absorption of starch or sucrose; and has been widely known as an agent for the prophylaxis or treatment of diabetes.

Further, many patients suffering from the diabetes also superinduce hyperlipemia, hypertension, and other diseases, thus treatment of diabetes itself as well as treatment of such complications have been left as an important medical or pharmaceutical problem.

There have been known to use the α-glucosidase inhibitor alone, in addition, to use the α-glucosidase inhibitor in combination with a variety of drugs. For example, there have been known (1) an obesity drug comprising acarbose, one of an α-glucosidase inhibitor, in combination with a lipase inhibitor (e.g. , see Patent Document 7), (2) an agent for the prophylaxis or treatment of diabetes or diabetes complications, comprising acarbose, voglibose, and miglitol, in combination with an insulin sensitivity enhancer (e.g., see Patent Document 8), (3) an agent for the prophylaxis or treatment of type II diabetes, comprising acarbose, voglibose, or miglitol, in combination with deoxyfructosazine (e.g., see Patent Document 9), and (4) an agent for the prophylaxis or treatment of diabetes, comprising acarbose, voglibose, or miglitol, in combination with a non-sulfonylurea insulin secretagogue (e.g., see Patent Document 10).

However, any pharmaceutical composition has not been known which combines the α-glucosidase inhibitor with a fibrate compound or HMG-CoA inhibitor (e.g., a statin compound) of the present invention.

Thus, there is a demand for a sufficiently excellent pharmaceutical composition having an excellent prophylactic and/or therapeutic effect as pharmaceuticals on metabolic syndrome, hyperlipemia, diabetes, diabetes complications and other diseases, as well as reducing the side effects.
Patent Document 1: JP-2002-502869A
Patent Document 2: JP-2002-533410A
Patent Document 3: JP-2002-533413A
Patent Document 4: JP-2003-528928A
Patent Document 5: JP-2001-294537A
Patent Document 6: JP-2001-514224A
Patent Document 7: JP-2780932B
Patent Document 8: JP-3148973B
Patent Document 9: WO01/47468
Patent Document 10: JP-2001-316293A

### DISCLOSURE OF THE INVENTION

### PROBLEMS TO BE SOLVED BY THE INVENTION

It is therefore an object of the present invention to provide a pharmaceutical composition having an excellent prophylactic and/or therapeutic effect on target diseases (e.g., metabolic syndrome, hyperlipemia, diabetes, and diabetes complications); use of a hyperlipidemic agent and an α-glucosidase inhibitor for preparing the pharmaceutical composition (a pharmaceutical preparation); and a prophylactic and/or therapeutic method for the target diseases.

It is another object of the present invention to provide a pharmaceutical composition having an excellent prophylactic and/or therapeutic effect on target disease even if using an α-glucosidase inhibitor in a small amount as well as being capable of suppressing (or reducing) side effects of the α-glucosidase inhibitor in spite of using the α-glucosidase inhibitor; use of a hyperlipidemic agent and an α-glucosidase inhibitor for preparing the pharmaceutical composition (a pharmaceutical preparation); and a prophylactic and/or therapeutic method for the target diseases.

### MEANS TO SOLVE THE PROBLEMS

The inventors of the present invention made intensive studies regarding various substances in order to find a pharmaceutical composition effective on the above-mentioned metabolic syndrome, hyperlipemia, diabetes, diabetes complications, and finally found that a pharmaceutical composition (combined pharmaceutical composition) which combines a fibrate compound and/or a statin compound with an α-glucosidase inhibitor as active ingredients (hereinafter, sometimes refers as "active component", or "effective ingredient" or "effective component") has an excellent therapeutic effect in a conventional dose or a smaller dose, and is capable of reducing side effects of the α-glucosidase inhibitor. The present invention was accomplished based on the above findings. As mentioned above, such a combined pharmaceutical composition would be never motivated from such a known example in which a hyperlipidemic agent (such as a fibrate compound or a statin compound), or an α-glucosidase inhibitor is individually used; or a known example in which either the hyperlipidemic agent or the α-glucosidase inhibitor is used in combination with other drugs.

That is, the pharmaceutical composition (combined pharmaceutical composition) of the present invention is a combined pharmaceutical composition including a combination (a) at least one hyperlipidemic agent selected from the group consisting of a fibrate compound and an HMG-CoA reductase inhibitor and (b) an α-glucosidase inhibitor, wherein the pharmaceutical composition is
(i) a pharmaceutical composition comprising the hyperlipidemic agent (a) and the α-glucosidase inhibitor (b), or
(ii) a pharmaceutical combination including a pharmaceutical component comprising the hyperlipidemic agent (a) and a pharmaceutical component comprising the α-glucosidase inhibitor (b).

Among the hyperlipidemic agents (a), the fibrate compound may be at least one member selected from the group consisting of fenofibrate, bezafibrate, clinofibrate, clofibrate, simfibrate, fenofibric acid, and gemfibrozil, or a salt thereof. The HMG-CoA reductase inhibitor may be at least one statin compound selected from the group consisting of pravastatin, simvastatin, fluvastatin, atorvastatin, lovastatin, cerivastatin, pitavastatin, and rosvastatin, or a salt thereof. The α-glucosidase inhibitor (b) may be at least one member selected from the group consisting of voglibose, acarbose, miglitol, and emiglitate, or a salt thereof. In the combined pharmaceutical composition, the proportion of the α-glucosidase inhibitor (b) may be about 0.001 to 50 parts by weight (e.g., about 0.01 to 10 parts by weight) relative to 100 parts by weight of the hyperlipidemic agent (a). The combined pharmaceutical composition of the present invention also includes a pharmaceutical composition comprising fenofibrate and voglibose in combination, which is (i) a pharmaceutical composition containing the fenofibrate and the voglibose, or (ii) a pharmaceutical combination including a pharmaceutical component containing the fenofibrate in combination with a pharmaceutical component containing the voglibose.

The pharmaceutical composition (combined pharmaceutical composition) of the present invention may be an agent for the prophylaxis or treatment of metabolic syndrome. Moreover, the combined pharmaceutical composition may be an agent for the prophylaxis or treatment of at least one symptom (or disease) selected from the group consisting of hyperlipemia, diabetes, diabetes complications, a symptom of hyperglycemia after a meal in diabetics, impaired glucose tolerance (IGT), decrease of glucose tolerance, hypertension, hyperinsulinemia, hyperammonemia, obesity or a complication thereof, fatty liver, and hepatitis.

The combined pharmaceutical composition may be (i) a pharmaceutical preparation containing (a) a hyperlipidemic agent and (b) an α-glucosidase inhibitor, or (ii) a pharmaceutical combination including a pharmaceutical preparation containing the hyperlipidemic agent (a) and a pharmaceutical preparation containing the α-glucosidase inhibitor (b).

The present invention also includes use of (a) at least one hyperlipidemic agent selected from the group consisting of a fibrate compound and an HMG-CoA reductase inhibitor, and (b) an α-glucosidase inhibitor for producing a pharmaceutical preparation.

Moreover, the present invention also includes a combined pharmaceutical composition reducing side effects or dose of an α-glucosidase inhibitor, which includes a combination of (a) at least one hyperlipidemic agent selected from the group consisting of a fibrate compound and an HMG-CoA reductase inhibitor with (b) an α-glucosidase inhibitor, wherein the combined pharmaceutical composition is (i) a pharmaceutical composition containing the hyperlipidemic agent (a) and the α-glucosidase inhibitor (b), or (ii) a pharmaceutical combination including a pharmaceutical component containing the hyperlipidemic agent (a) with a pharmaceutical component containing the α-glucosidase inhibitor (b).

In the prophylactic or therapeutic method of the present invention, by administering (a) at least one hyperlipidemic agent selected from the group consisting of a fibrate compound and an HMG-CoA reductase inhibitor and (b) an α-glucosidase inhibitor to human or non-human animals, target diseases (e.g., at least one symptom selected from the group consisting of metabolic syndrome, hyperlipemia, diabetes, diabetes complications, a symptom of hyperglycemia after a meal in diabetics, impaired glucose tolerance (IGT), decrease of glucose tolerance, hypertension, hyperinsulinemia, hyperammonemia, obesity or a complication thereof, fatty liver, and hepatitis) are prevented or treated.

Incidentally, in the present description, a pharmaceutical composition (or a pharmaceutical preparation) embraces a meaning including pharmaceuticals (or a medical product, drugs, remedy or agent), a quasi drug, and the like.

### EFFECTS OF THE INVENTION

In the combined pharmaceutical composition of the present invention, since a fibrate compound and/or an HMG-CoAreductase inhibitor (e.g., a statin compound) useful as a hyperlipidemic agent is combined with an α-glucosidase inhibitor which is a therapeutic agent for diabetes, diabetes complications, and the like, a prophylactic and/or therapeutic effect on target symptoms (diseases) (e.g., metabolic syndrome, hyperlipemia, diabetes, diabetes complications, a symptom of hyperglycemia after a meal in diabetics, impaired glucose tolerance (IGT), decrease of glucose tolerance, hypertension, hyperinsulinemia, hyperammonemia, obesity or a complication thereof, fatty liver, and hepatitis) is enormously increased or improved. Moreover, compared with the case to use each of the hyperlipidemic agent and the α-glucosidase inhibitor alone, the prophylactic and/or therapeutic effect (e.g. , effects such as reducing blood sugar level, serum total cholesterol, and blood-triglyceride level) is greatly improved. Therefore, even if the active ingredient (particularly, an α-glucosidase inhibitor) is administered in a conventional dose (an amount to be employed), the active ingredient is attributed to an excellent prophylactic and/or therapeutic effect as well as to dose reduction. Thereby, the present invention realizes to provide a pharmaceutical product with high safety as well as with few side effects of the α-glucosidase inhibitor.

### DETAILED DESCRIPTION OF THE INVENTION

The pharmaceutical composition of the present invention is a pharmaceutical composition including a combination of (a) at least one hyperlipidemic agent selected from the group consisting of a fibrate compound and a hydroxymethylglutaryl (HMG)-CoA reductase inhibitor with (b) an α-glucosidase inhibitor.

Among the hyperlipidemic agents used in the present invention, the fibrate compound may sufficiently be a lipid-improving component or drug, particularly a component or drug reducing an amount of the blood-triglyceride and/or blood-cholesterol and others. For example, the fibrate compound may be a compound showing an action of improving lipid level by inhibiting synthesis or secretion of triglyceride in the liver and activating a lipoprotein lipase. The fibrate compound may include, for example, fenofibrate, bezafibrate, clinofibrate, clofibrate, simfibrate, fenofibric acid, gemfibrozil, salts thereof (e.g., aluminium clofibrate), and others.

The fibrate compounds may be used singly or in combination. Among the fibrate compounds, in particular, the preferred one includes fenofibrate, and bezafibrate, or a salt thereof (e.g., a physiologically or pharmaceutically acceptable salt).

Among the therapeutic agents of hyperlipemia used in the present invention, the HMG-CoA reductase inhibitor may sufficiently be a component or drug inhibiting hydroxymethylglutaryl-CoA (HMG-CoA) reductase and having a lipid-improving property, particularly lowering an amount of a blood-cholesterol. As such an HMG-CoA reductase inhibitor, there may be used a statin compound, and others. Examples of the statin compound may include pravastatin, simvastatin, fluvastatin, atorvastatin, lovastatin, cerivastatin, pitavastatin, rosvastatin, salts thereof, and others. The HMG-CoA reductase inhibitor may be used singly or in combination. Among these HMG-CoA reductase inhibitors, in particular, the preferred one includes pravastatin, and atorvastatin, or a salt thereof (e.g., a physiologically or pharmaceutically acceptable salt).

Incidentally, the fibrate compound and HMG-CoA reductase inhibitor (e.g., a statin compound) also encompass a derivative of each compound (e.g., an ester) or a prodrug.

As the salts of the fibrate compound and statin compound (e.g., a physiologically or pharmaceutically acceptable salt), there may be exemplified a salt of the fibrate compound or statin compound with an inorganic or organic base, a salt thereof with an inorganic or organic acid, a salt thereof with a neutral, basic, or acidic amino acid, and other salts. The preferred examples of a salt with an inorganic base include a salt with an alkali metal such as sodium and/or potassium, a salt with an alkaline earth metal such as calcium or magnesium, a salt with aluminum and/or ammonium, and the like. The preferred examples of a salt with organic base include a salt with an alkylamine such as trimethylamine or triethylamine; a salt with a heterocyclic amine such as pyridine and picoline; a salt with an alkanolamine such as ethanolamine, diethanolamine, or triethanolamine; a salt with a cycloalkylamine such as dicyclohexylamine; a salt with an alkylenediamine derivative such as N,N-dibenzylethylenediamine; and others. The preferred examples of a salt with an inorganic acid include a salt with hydrochloric acid, hydrobromic acid, nitric acid, sulfuric acid, phosphoric acid, and others. The preferred examples of a salt with an organic acid include, for example, a salt with a monocarboxylic acid such as formic acid, acetic acid, and trifluoroacetic acid; a salt with a polycarboxylic acid such as fumaric acid, maleic acid, and oxalic acid; a salt with a hydroxycarboxylic acid such as tartaric acid, citric acid, succinic acid, and malic acid; a salt with a sulfonic acid such as methanesulfonic acid, benzenesulfonic acid, and p-toluenesulfonic acid, and others. The preferred examples of a salt with a neutral amino acid include a salt with glycine, valine, or leucine. The preferred examples of a salt with a basic amino acid include a salt with arginine, lysine, or ornithine. The preferred examples of a salt with an acidic amino acid include a salt with aspartic acid or glutamic acid. The hyperlipidemic agents may be used singly or in combination.

The α-glucosidase inhibitor used in the present invention may be a drug having an action delaying digestion and absorption of starch or sucrose by inhibiting a digestive enzyme such as amylase, maltase, an α-dexitrinase, and sucrase. Examples of such an α-glucosidase inhibitor may include voglibose, acarbose, miglitol, emiglitate, salts thereof, and the like. Examples of the salts thereof may include the salts exemplified in the paragraph of the salt of the fibrate compound (e.g., a physiologically or pharmaceutically acceptable salt), and others. Incidentally, the α-glucosidase inhibitor may also include a derivative (e.g., an ester) or prodrug of the above-exemplified α-glucosidase inhibitor. These α-glucosidase inhibitors may be used singly or in combination. Among the α-glucosidase inhibitors, voglibose, acarbose, and the like are particularly preferred.

Incidentally, the active ingredients such as the hyperlipidemic agent and the α-glucosidase inhibitor may be an optically active substance or a racemic body.

In the pharmaceutical composition (or a pharmaceutical preparation) of the present invention, the proportion of the α-glucosidase inhibitor relative to 100 parts by weight of the hyperlipidemic agent may be selected from the range of about 0.001 to 100 parts by weight, and is, for example, about 0.001 to 50 parts by weight (e.g., about 0.05 to 20 parts by weight), preferably about 0.01 to 10 parts by weight, and more preferably about 0.05 to 5 parts by weight. Incidentally, in the case of using the fibrate compound and the HMG-CoA reductase inhibitor in combination, the proportion (weight ratio) of the fibrate compound relative to the HMG-CoA reductase inhibitor [the fibrate compound/the HMG-CoA reductase inhibitor] may be about 10/90 to 90/10, preferably about 20/80 to 80/20, and more preferably about 30/70 to 70/30.

The combination of the hyperlipidemic agent with the α-glucosidase inhibitor may include a combination of the fibrate compound with the α-glucosidase inhibitor, a combination of the HMG-CoA reductase inhibitor (e.g., a statin compound) with the α-glucosidase inhibitor, and a combination of the fibrate compound and an HMG-CoA reductase inhibitor with the α-glucosidase inhibitor. Among these combinations, in particular, the preferred combination includes a combination of fenofibrate and/or bezafibrate with voglibose (particularly, combination of fenofibrate with voglibose), a combination of fenofibrate and/or bezafibrate with acarbose, a combination of pravastatin and/or atorvastatin with voglibose, a combination of pravastatin and/or atorvastatin with acarbose, and others.

In the combined pharmaceutical composition of the present invention, (a) the hyperlipidemic agent (the fibrate compound and/or HMG-CoA reductase inhibitor) may be sufficiently used in combination with (b) the α-glucosidase inhibitor. The combined pharmaceutical composition may be (i) a pharmaceutical composition (a pharmaceutical preparation) comprising both the hyperlipidemic agent (a) and the α-glucosidase inhibitor (b); or (ii) a pharmaceutical combination (a pharmaceutical preparation) including a combination of a pharmaceutical component (a pharmaceutical preparation) comprising the hyperlipidemic agent (a) with a pharmaceutical component (a pharmaceutical preparation) comprising the α-glucosidase inhibitor (b). In the combined pharmaceutical composition, as the pharmaceutical composition, the hyperlipidemic agent (a) and the α-glucosidase inhibitor (b), both are the active ingredients, may be used without any bases or substrates as they are. Moreover, the hyperlipidemic agent (a) and the α-glucosidase inhibitor (b) may be suitably used for a pharmaceutical preparation (or a production thereof), and is usually used as a form (or mode) of a pharmaceutical preparation in many cases. Incidentally, the present invention also includes a kit including a combination of a pharmaceutical component (or a pharmaceutical preparation) containing the hyperlipidemic agent (a) with a pharmaceutical component (or a pharmaceutical preparation) containing the α-glucosidase inhibitor (b).

Incidentally, the mode (manner) of administration of the pharmaceutical combination (ii) is not particularly limited to a specific one. For example, a pharmaceutical component (or a pharmaceutical preparation) containing the hyperlipidemic agent (a) and a pharmaceutical component (or a pharmaceutical preparation) containing the α-glucosidase inhibitor (b) may be administrated together (simultaneously) or individually (separately). In the separate administration of two pharmaceutical components (or pharmaceutical preparations), after administering one pharmaceutical component (or a pharmaceutical preparation) to a subject, the other pharmaceutical component may be administered to the same subject in a time-staggered manner (after some interval of time). The other pharmaceutical component may be usually administered during the period when the effect of precedently administered active ingredient (component) is sustained practically. In the case of separate administration of the hyperlipidemic agent and the α-glucosidase inhibitor, in order to lower the amount of the active ingredients or to effectively reduce the side effects of the α-glucosidase inhibitor, it is preferred to administer the both pharmaceutical components simultaneously, or administer one pharmaceutical component immediately after administering the other component. Incidentally, prior to the administration (e.g., just before administration), a pharmaceutical component (or a pharmaceutical preparation) comprising the hyperlipidemic agent and a pharmaceutical component (or a pharmaceutical preparation) comprising the α-glucosidase inhibitor may be mixed (if necessary, mixed with a diluent), and administered as a mixture.

The active ingredients (the hyperlipidemic agent (a) and/or the α-glucosidase inhibitor (b)) may be usually mixed with a physiologically acceptable carrier, excipient, binder, diluent and/or the like to make formulation or preparation, and administered as a pharmaceutical preparation (a pharmaceutical composition) practically.

The dosage form of the pharmaceutical composition (or pharmaceutical preparation) is not particularly limited to a specific one, and may be any one of a liquid preparation (e.g., a suspension, an emulsion, a syrup, an injection, a jelly, and a gumi), a semi-solid preparation (e.g., an ointment such as a soft ointment, or a hard ointment), a solid preparation [e.g., a fine powder, a subtle granule, a powder, a granule, a pill (ball), a capsule (e.g., a hard capsule, and a soft capsule), a tablet, an compression preparation, and a fused and solidified preparation].

Moreover, the mode (or manner) of administration of the pharmaceutical composition of the present invention is not particularly limited to a specific one, and may be any form of oral administration or non-oral administration. Among the pharmaceutical compositions, the oral preparation may include, for example, a granule (including a dry syrup), a powder, a tablet (including a buccal, an oral disintegrant, a lozenge (trochiscuss), and a chewable tablet), a capsule (including a soft capsule and a microcapsule), a syrup, an emulsion, a suspension, a jelly, a gumi, and others. Incidentally, the oral preparation may also include a preparation controlling release of the active ingredients in the body with a known preparation component (e.g., an immediate (rapid) release preparation, and a sustained release preparation). Moreover, examples of the non-oral preparation may include an injection (including a subcutaneous injection, an intravenous injection, an intramuscular injection, an intraperitoneal injection, and a drip infusion), an external preparation (including a nasal spray preparation, a transdermal preparation, an ointment, and a suppository), and others.

The pharmaceutical preparation comprising the active ingredients, independent of the administration routes, can be formulated (or produced) in a conventionally used procedure by combining the active ingredients with a carrier (a preparation additive suitable for the pharmaceutical preparation (sometimes referred to as "preparation component")). That is, the pharmaceutical preparation, for example, may be manufactured in accordance with a process for preparing (producing) a tablet, a granule, a powder, a hard capsule, a soft capsule, a lozenge, a dry syrup, a syrup, a liquid preparation and a suspension described in Japanese Pharmacopoeia. Incidentally, the solid preparation may be prepared with at least one carrier (particularly at least an excipient) selected from the group consisting of a binder, an excipient, and a disintegrator. For example, the granule may be usually prepared by granulating the active ingredients and a carrier component with the use of an extrusion granulation or a spray granulation, and if necessary sizing. The tablet may be manufactured by mixing the granulate, if necessary, with an additive, compression-molding the mixture, and where necessary, coating the compression-molded substance with a per se known method for imparting a taste-masking property, an enteric property, or a prolonged (sustained) release property. The capsule may be prepared by filling a granule into a capsule. Moreover, the powdery solid preparation such as a fine powder (e.g., a powdery external preparation) may be prepared by mixing the active ingredients with an excipient, and if necessary a thickener. Incidentally, the solid suppository may be prepared by mixing the active ingredients with a carrier component, and if necessary an additive, and compressing the mixture with a compressor to give a compressed preparation. Moreover, by cooling a melt-mixed mixture for solidification, a melt-solidified preparation may be prepared.

The liquid preparation (including a jelly and a gumi) may be prepared, depending on a dosage form, by mixing (e.g., dissolving, suspending (dispersing), and emulsifying) the active ingredients with a liquid carrier component (e.g., an aqueous solvent such as purified water, an oily solvent, a gel base (e.g., an aqueous or oily gel)), and if necessary an additive (e.g., an emulsifier, a dispersant or suspension, an isotonizing agent, a solubilizer, a preservative, a stabilizer, a flavoring substance, a pH control agent (pH regulator), and a buffer), and if desired, the mixture is sterilized. The ointment may be prepared by mixing or kneading the active ingredients with a carrier component (e.g., an oily base, an aqueous base) (and if necessary an additive), if desired under heating.

The carrier (preparation additive) may include an additive commonly used upon producing drugs and medicines in the above-mentioned dosage form. For example, the carrier may be suitably selected, depending on administration route and an application of the pharmaceutical composition or preparation from the components (e.g., an excipient, a binder, a disintegrator, lubricant, and a coating agent) described in the Japanese Pharmacopoeia, in addition, (1) Handbook for additives of drugs and medicines, Maruzen Co., Ltd., (1989), (2) Subject-book for additives of drugs and medicines, the first edition (The Yakuji Nippo Limited, published on January 14, 1994), (3) Addendum of Subject-book for additives of drugs and medicines, the first edition, The Yakuji Nippo Limited, (1995), and (4) Pharmaceutics, the revised fifth edition, Nankodo Co., Ltd. (1997).

Among carrier components or additives for the solid preparation, examples of the excipient may include a saccharide such as lactose, saccharose, glucose, mannitol, or sorbitol; a starch such as a corn starch; a polysaccharide such as a crystalline cellulose (including a microcrystalline cellulose), and others. Examples of the binder may include a polysaccharide such as a pregelatinized starch, an agar, a gum Arabic, or a dextrin; a synthetic polymer such as a polyvinylpyrrolidone, a polyvinyl alcohol, a carboxyvinyl polymer, or a polylactic acid; a cellulose ether such as a methyl cellulose, an ethyl cellulose, a carboxymethyl cellulose, a carboxymethyl cellulose sodium, a hydroxymethyl cellulose, or a hydroxypropyl cellulose, or a hydroxypropyl methyl cellulose; and others. Examples of the disintegrant may include a calcium carbonate, a carboxymethyl cellulose calcium (carmellose calcium), a crosslinked povidone, a low-substituted hydroxypropyl cellulose, and others. Examples of the lubricant may include a talc, magnesium stearate, a polyethylene glycol 6000, and the like. Moreover, the solid preparation may contain, as an additive(s), a disintegration aid, a surfactant (e.g., an anionic surfactant such as an sodium alkyl sulfate, a nonionic surfactant such as a polyoxyethylene sorbitan fatty acid ester or a polyoxyethylene fatty acid ester), a lipid (e.g., a fat and oil, such as a hydrogenated vegetable oil, and a phospholipid), a thickener (e.g., a natural gum, a cellulose derivative, and an acrylic acid polymer), an oily base [e.g., a higher fatty acid ester, for example, a glyceride of a higher fatty acid (including a cocoa butter and a witepsol); a medium chain fatty acid (including a Migriol); a vegetable oil (including a sesame oil, a soybean oil, and a cottonseed oil); a hydrocarbon-series base such as a petrolatum (vaseline), a liquid paraffin, or a wax; and a cetanol], an aqueous base (e.g., a hydrophilic vaseline, and macrogol), an antioxidant, a preservative or antiseptic agent, a wetting agent, an antistatic agent, and others. Incidentally, in the compressed preparation and the melt-solidified preparation (e.g., a solid suppository), the oily base and the aqueous base may be used as a carrier component in many cases.

Incidentally, as a coating agent used for the coating treatment, there may be used, for example, a saccharide, a cellulose derivative such as an ethyl cellulose or a hydroxymethyl cellulose, a polyoxyethylene glycol, a cellulose acetate phthalate, a hydroxypropyl methyl cellulose phthalate, an Eudragit (a copolymer of methacrylic acid and acrylic acid), and others. The coating agent may be an enteric component such as a hydroxypropyl methyl cellulose phthalate, or a gastric coating component comprising a polymer containing a basic component such as dialkylaminoalkyl(meth)acrylate (e.g., an Eudragit).

As a carrier component or additive used for the liquid preparation (including a jelly, a gumi, and the like), there may be mentioned an aqueous solvent (including a purified water such as distilled water, an alcoholic solvent such as ethanol, glycerin, propylene glycol, a polyethylene glycol (e.g., macrogol), in addition, a physiological saline, a Ringer's solution, and the like), an oily solvent (including a vegetable oil such as olive oil, sesame oil, cottonseed oil, or corn oil; and tricaprylin), a gel base or gelatinizing agent (e.g., a natural gum or polysaccharide (e.g., a pectin, a locust bean gum, a gum Arabic, a tragacanth gum, sodium alginate, an agar, a carrageenan, a hyaluronic acid, and a chondroitin sulfate), a cellulose derivative (e.g., cellulose ethers exemplified in the paragraph of the binder), a synthetic polymer (e.g., synthetic polymers (including a vinyl polymer) exemplified in the paragraph of the binder, in addition, a polyethylene glycol, an acrylic acid polymer such as a (meth)acrylic acid copolymer), a dispersant (e.g., Tween 80, a polyethylene glycol, a carboxymethyl cellulose, and sodium alginate), a suspension (e.g., a polysaccharide such as a gum Arabic or a locust bean gum, a cellulose ester such as a carboxymethyl cellulose, and a nonionic surfactant), the above-exemplified surfactants, an emulsifier, a solubilizing agent, a solubilizer (including sodium salicylate, and sodium acetate), an isotonizing agent (including sodium chloride, glycerine, sorbitol, glucose, and an invert sugar), a viscosity adjuster (e.g., the above-exemplified thickeners), a preservative or antiseptic agent (e.g., methyl paraben, propyl paraben, benzyl alcohol, chlorobutanol, phenol, sodium benzoate, p-hydroxybenzoate ester, and benzalkonium chloride), an antioxidant, a stabilizer (including a human serum albumin), the above-exemplified saccharides, a pH control agent (including an acid component such as carbonic acid, phosphoric acid, citric acid, or hydrochloric acid; and a base component such as sodium hydroxide), a buffer (e.g., an organic acid-series buffer such as sodium acetate, citric acid, sodium citrate, potassium bitartrate, or sodium bitartrate; a phosphate-series buffer such as sodium dihydrogen phosphate or potassium dihydrogen phosphate; a boric acid-series buffer such as boric acid or borax (or sodium borate)), and others. Incidentally, the liquid preparation such as an injection may contain a soothing agent (including benzalkonium chloride, procaine hydrochloride, etc.) as an additive.

As a carrier component or additive used for a semi-solid preparation, there may be mentioned, an oily base [e.g., a higher fatty acid ester, for example, a glyceride of a higher fatty acid (including a cocoa butter and a witepsol); a medium chain fatty acid (including a Migriol); the above-exemplified oily solvents such as a vegetable oil (including a sesame oil, a soybean oil, and a cottonseed oil); a hydrocarbon-series base such as a petrolatum, a liquid paraffin, or a wax; and a cetanol], an aqueous base (e.g., a hydrophilic vaseline, and macrogol), various additives exemplified in the paragraphs of the solid preparation and the liquid preparation (e.g., an aqueous solvent, a pH control agent, a buffer, a preservative or antiseptic agent, and an antioxidant), and others.

Incidentally, these pharmaceutical preparations may contain a flavoring substance (e.g., a sweetening agent) or a masking agent, a colorant, an odor improving agent (e.g., an aromatic substance), a ref rigerant , an antifoaming agent, and others.

The pharmaceutical composition (or pharmaceutical preparation) of the present invention may contain, if necessary, other physiologically active ingredient or pharmacologically active ingredient, for example, a vitamin, an amino acid, an antacid, a crude drug component, an enzyme, an antihypertensive agent, other therapeutic agents of hyperlipemia (e.g., nicotine acid or a derivative thereof, an ion-exchanging agent, a probucol, and a phytosterol), a diabetic drug (e.g., an insulin preparation, and a hypoglycemic drug), and others.

Incidentally, the pH of the liquid preparation may be, for example, usually about 4 to 9, preferably about 5 to 8.5, and more preferably about 5.5 to 8.5.

The pharmaceutical composition of the present invention is useful for an agent (therapeutic product) for the prophylaxis and/or treatment of metabolic syndrome. Herein, metabolic syndrome is a symptom (disease) substantially diagnosed based on four risk factors, that is, abnormal sugar (saccharide) metabolism, abnormal lipid metabolism, obesity, and hypertension, and a patient with metabolic syndrome may fall under either two diagnostic criteria proposed below.
(1) The diagnostic criteria in U.S. (Adult Treatment Panel III; ATP III)
   (a) Visceral obesity
      Waist circumference: Men > 102 cm, Women > 88 cm
   (b) Hyperglyceridemia
      Triglyceride ≥ 150 mg/dl
   (c) Hypo-HDL-cholesterolemia
      HDL cholesterol: Men < 40 mg/dl, Women < 50 mg/dl
   (d) Hypertension
      Blood pressure ≥ 130/85 mmHg
   (e) Fasting hyperglycemia
      Fasting glucose ≥ 110 mg/dl
      ATP III defines metabolic syndrome as involving three or more of the above factors (a) to (e).
(2) The diagnostic criteria in World Health Organization (WHO)
   (A) Type 2 diabetes
   (B) Abnormal sugar (saccharide) metabolism (boundary type)
   (C) Insulin resistance (insulin sensitivity by glucose-clamping is within a low-level of 25%)
      Fulfill at least one criterion from (A) to (C) (I)
      (a) Hypertension
         Systolic blood pressure ≥ 160 mmHg or diastolic phase of uterine contraction ≥ 90 nnHg
      (b) Obesity
         Degree of obesity ≥ 30; or waist/hip ratio: Men > 0.9, Women > 0.85
      (c) Abnormal lipid metabolism
         Triglyceride ≥ 150 mg/dl; or HDL cholesterol: Men < 35 mg/dl, Women < 39 mg/dl
      (d) A slight amount of albumin
         Excretion rate of albumin ≥ 20
Fulfill at least two criteria from (a) to (d) (II)
The case of fulfilling both (I) and (II) is designated (defined) as metabolic syndrome.

Further, more specifically, the pharmaceutical composition of the present invention may be used as an agent for the prophylaxis and/or treatment of the metabolic syndrome, in addition, for example, symptoms such as diabetes (e.g., Type 1 diabetes, Type 2 diabetes, and pregnancy diabetes), diabetes complications (e.g., retinopathy, nephropathy, diabetic neuropathy, and macroangiopathy), a symptom of hyperglycemia after a meal in diabetics, impaired glucose tolerance (IGT), an agent for suppressing development of impaired glucose tolerance into diabetes, decrease of glucose tolerance, a cardiovascular disease [e.g., hyperlipidemia (e.g., hypertriglyceridemia, hypercholesterolemia, and hypo-high-density-lipoproteinemia), and hypertension], hyperinsulinemia, coronary and cerebrovascular disorder, hyperammonemia, hyperammonemia, hyperuricemia, obesity or a complication thereof, a bone metabolism disorder (e.g., osteoporosis, and osteopenia), fatty liver, hepatitis, dumping syndrome, and glycogenosis. Incidentally, the pharmaceutical composition of the present invention is suitable for the prophylaxis and/or treatment of at least one symptom selected from above symptoms , and also is useful as an agent for the prophylaxis and/or treatment of a plurality of symptoms. In particular, the pharmaceutical composition of the present invention is useful as an agent for the prophylaxis and/or treatment of at least one symptom selected from the group consisting of metabolic syndrome, hyperlipemia, diabetes, diabetes complications, a symptom of hyperglycemia after a meal in diabetics, impaired glucose tolerance (IGT), decrease of glucose tolerance, hypertension, hyperinsulinemia, hyperammonemia, obesity or a complication thereof, fatty liver, and hepatitis.

Since the pharmaceutical composition of the present invention has been already used as a pharmaceutical agent by itself, the pharmaceutical composition is low in toxicity, and its safety has been established. The pharmaceutical composition can be safely used in human and non-human animals, usually, mammals (e.g. humans, mice, rats, rabbits, dogs, cats, bovines, horses, swines (pigs), and monkeys). Accordingly, in the present invention, the hyperlipidemic agent (a) and the α-glucosidase inhibitor (b) can be administered to such a human or non-human animal.

The dose of the pharmaceutical composition of the present invention may be suitably determined in accordance with the conventional doses in the individual drugs and can be suitably selected depending on the subject to be administered, the age and body weight of the subject, the symptom, the administration time, the dosage form, the method of administration, and others. In the present invention, the dose of the hyperlipidemic agent (a fibrate compound and/or HMG-CoA reductase inhibitor) and that of the α-glucosidase inhibitor can be suitably selected with reference to the clinically employed dose respectively. More concretely, the object of the present invention can be achieved by administering such a clinically conventional dose or smaller dose (e.g., a dose being about one-half to one-fifth of the conventional dose). For example, the oral daily dose of the fibrate compound for an adult is about 20 to 400 mg, and preferably about 30 to 350 mg. The oral daily dose of the HMG-CoA reductase inhibitor (e.g., a statin compound) for an adult is 0.5 to 50 mg, and preferably about 1 to 45 mg. Moreover, the oral daily dose of the α-glucosidase inhibitor for an adult can be selected from the range of, for example, about 0.01 to 1 mg, and preferably about 0.03 to 0.8 mg for voglibose; about 50 to 300 mg, and preferably about 70 to 250 mg for acarbose. The frequency of administration is not particularly limited to a specific one, for example, may be one time a day, or if necessary a several times (e.g., 2 to 4 times) a day.

Incidentally, in the pharmaceutical preparation, the compounding (blending) ratio of each active ingredients may be suitably selected depending on the subject to be administered, the age and body weight of the subject, the symptom, the administration time, the dosage form, the method of administration, the necessary amount of the active ingredients and a combination thereof, and others. More specifically, it is preferred to prepare a desired preparation having a compounding ratio in which the above amount to be clinically used is taken into consideration.

The combined pharmaceutical composition of the present invention has a synergetic (enhancing) effect compared with the single administration of each of the active ingredients (each of a hyperlipidemic agent and an α-glucosidase inhibitor) alone. For example, in a diabetic model rat, compared with administering individually each of the two active ingredients alone, the pharmaceutical composition of the present invention effectively lowers blood sugar level in diabetics and, therefore, is applicable to the prophylaxis and/or treatment of diabetes and the diabetic complications. Accordingly, the pharmaceutical composition of the present invention can reduce a dose of active ingredients (particularly an α-glucosidase inhibitor). Moreover, since the pharmaceutical composition of the present invention exerts sufficient effects in a smaller dose as compared with the dose of each of the active ingredients alone, the side effects of the α-glucosidase inhibitor (e.g., an impaired liver function such as fulminant hepatitis, an impaired digestive organ such as diarrhea or constipation, and anemia) can be reduced (or lowered).

### INDUSTRIAL APPLICABILITY

The pharmaceutical composition of the present invention much more effectively lowers blood sugar level in diabetics than an α-glucosidase inhibitor alone does, and, therefore, is applicable to the prophylaxis and treatment of diabetes and the diabetic complications. Moreover, since the pharmaceutical composition of the present invention exerts sufficient effects with a conventional or smaller dose compared with cases administering each drug (pharmaceutical component) alone, the side effects of the drug can be reduced. The pharmaceutical composition of the present invention is useful not only as an agent for the prophylaxis and/or treatment of the above-mentioned diabetes and diabetes complications, but also as an agent for the prophylaxis and/or treatment of syndromes, for example, metabolic syndrome, hyperlipemia, a symptom of hyperglycemia after a meal in diabetes, impaired glucose tolerance (IGT), decrease of glucose tolerance, hypertension, hyperinsulinemia, hyperammonemia, obesity or a complication thereof, fatty liver, or hepatitis.

### EXAMPLES

The following examples are intended to describe this invention in further detail and should by no means be interpreted as defining the scope of the invention.

| Example 1 (Tablet) | |
|---|---|
| Fenofibrate | 100.0 mg |
| Voglibose | 0.2 mg |
| Lactose | 69.2 mg |
| Microcrystalline cellulose | 29.6 mg |
| Magnesium stearate | 1.0 mg |
| Total weight | 200.0 mg |

| Example 2 (Tablet) | |
|---|---|
| Pravastatin | 10.0 mg |
| Voglibose | 0.2 mg |
| Lactose | 132.2 mg |
| Microcrystalline cellulose | 56.6 mg |
| Magnesium stearate | 1.0 mg |
| Total weight | 200.0 mg |

Test Example 1. Effect of combination use of fenofibrate and voglibose on a streptozotocin-induced diabetic model

### <Test method>

Streptozotocin (STZ) (45 mg/kg) was dissolved in physiological saline, and the solution was intravenously administered to male Wister rats. At 13 days after administration, blood was collected from the rats, and blood sugar (blood glucose) level, serum total cholesterol and blood-triglyceride were measured. The rats were then divided into the following three groups, (i) administration of voglibose alone (0.2 mg/kg), (ii) administration of fenofibrate alone (50 mg/kg), and (iii) combined administration of both agents (doses of each agent were the same with above), and were repetitively (repeatedly) orally administered (by mouth). At 23 to 25 days after streptozotocin administration, blood was collected from the rats, and the parameters same as the above-mentioned were measured, and the measured values were compared and investigated.

### <Results>

Combination use (administration) of fenofibrate and voglibose significantly reduced each parameter compared with use of each drug (active ingredient) alone. The similar effects were also observed in the combination use of bezafibrate and voglibose.

Test Example 2. Effect of combination use of pravastatin and voglibose on a streptozotocin-induced diabetic model

### <Test method>

Streptozotocin (STZ) (45 mg/kg) was dissolved in physiological saline, and the solution was intravenously administered to male Wister rats. At 13 days after administration, blood was collected from the rats, and blood sugar level, serum total cholesterol and blood-triglyceride level were measured. The rats were then divided into the following 3 groups, (i) administration of voglibose alone (0.2 mg/kg), (ii) administration of pravastatin alone (10 mg/kg), and (iii) combined administration of both agents (doses of each agent were the same with above), and were repetitively orally administered. At 23 to 25 days after streptozotocin administration, blood was collected from the rats, and the parameters same as the above-mentioned were measured, and the measured values were compared and investigated.

### <Results>

Combination use of fenofibrate and voglibose significantly reduced each parameter compared with use of each drug alone.

As shown above, in the pharmaceutical composition of the present invention, the action caused by each single drug can be enhanced and the dose of each drug can be decreased (or lowered). As a result, the side effects caused by each single ingredient can be also reduced.

Test Example 3. Effect of combination use of fenofibrate and voglibose on a streptozotocin-induced diabetic model
Streptozotocin (STZ) was dissolved in physiological saline, and the solution was intravenously administered to male Wister rats at a dose of 45 mg/kg in terms of STZ (STZ-administered day: Day 0). At 13 days after administration (Day 13), blood was collected from the rats, and plasma glucose (GLU) concentration was measured. Based on the GLU concentration, STZ-treated rats were divided into 6 groups (10 rats/group). From Day 14 (14 days after STZ administration), test articles shown in Table 1 were orally administered to the rats once a day for 9 days. At 23 days after STZ administration (Day 23), the GLU concentration was measured (previous value: GLU concentration before sucrose loading) (incidentally, the test articles were not administered on Day 23). At 1 day after previous value measurement (Day 24), the test articles (test substances) shown in Table 1 were administered to the rats, and immediately after the administration, sucrose was orally administered at a dose of 2.5 g/kg to carry out sucrose loading. At 60 minutes after sucrose loading, blood was collected from the rats, and the GLU concentration (GLU concentration after sucrose loading) was measured.

Incidentally, as a control, a group in which neither STZ treatment nor test article administration were not conducted (Group 1), and a group in which after STZ treatment test article administration was not conducted (Group 2) were employed, and GLU concentrations before and after sucrose loading were also measured in the same manner with above.

Table 1 shows the GLU concentrations (g/L) before and after sucrose loading and the rate (%) of the GLU concentration change after loading relative to the GLU concentration before loading. Incidentally, each of the test articles was administered in the following dose a time: metformin (50 mg/kg), voglibose (0.2 mg/kg), and fenofibrate (50 mg/kg). Moreover, "N" represents the number of rats for determining the GLU concentration in the Table.

[Table 1]

**Table 1**

| Group | Pretreatment | Test articles | GLU concentration before loading (g/L) | GLU concentration after loading (g/L) | Rate of Change (%) | N |
|---|---|---|---|---|---|---|
| 1 | - | - | 1.30 ± 0.03 | 1.36 ± 0.03 | 104.6 | 8 |
| 2 | STZ | - | 4.35 ± 0.34 | 5.21 ± 0.30 | 119.8 | 10 |
| 3 | STZ | Metformin | 4.57 ± 0.36 | 5.41 ± 0.38 | 118.4 | 10 |
| 4 | STZ | Voglibose | 4.30 ± 0.47 | 4.50 ± 0.42 | 104.7 | 9 |
| 5 | STZ | Fenofibrate | 4.26 ± 0.55 | 4.78 ± 0.34 | 112.2 | 9 |
| 6 | STZ | Metformin Fenofibrate | 4.43 ± 0.27 | 5.03 ± 0.28 | 113.5 | 10 |
| 7 | STZ | Voglibose Fenofibrate | 4.83 ± 0.25 | 4.28 ± 0.37 | 88.6 | 8 |

As apparent from Table 1, the blood sugar level was increased about 20% by the sucrose loading in the STZ-treated group (Group 2), but the increase of blood sugar level was inhibited by voglibose administration (Group 4) to the same degree as that in the STZ-untreated control (Group 1). The blood sugar levels were also increased in Group 3 (administration of metformin alone), Group 5 (administration of fenofibrate alone), and Group 6 (administration of metformin and fenofibrate). On the contrary of these results, in Group 7 (administration of voglibose and fenofibrate), the blood sugar level was not increased, but decreased 11.4% compared with the previous value.

Test Example 4. Effect of combination use of fenofibrate and voglibose on a streptozotocin-induced diabetic model
Streptozotocin (STZ) was dissolved in physiological saline, and the solution was intravenously administered to male Wister rats at the dose of 45 mg/kg in terms of STZ (STZ-administered day: day 0). At 13 days after administration, blood was collected from the rats, and plasma glucose (GLU) concentration was measured. Based on the GLU concentration, STZ-treated rats were divided into 4 groups (7 rats/group). Incidentally, 3 groups out of the divided 4 groups were treated as a voglibose-administered group (Group 3), a fenofibrate-administered group (Group 4), and a voglibose- and fenofibrate-administered group (Group 5). From Day 14 (14 days after STZ administration), each of the test articles was orally administered to the rats of these Groups 3 to 5 once a day for 9 days. At 23 days after STZ administration (Day 23), the GLU concentration was measured (previous value: GLU concentration before sucrose loading) (incidentally, test articles were not administered on Day 23). At 1 day after previous value determination (Day 24), each of the test articles was administered to the rats of Groups 3 to 5, and immediately after the administration, sucrose was orally administered at a dose of 2.5 g/kg to carry out sucrose loading. At 60 minutes after sucrose loading, blood was collected from the rats, and the GLU concentration (GLU concentration after sucrose-loading) was measured. Incidentally, the test articles were administered to the rats of Groups 3 to 5 at the following dose a time.

Voglibose-administered group (Group 3):
voglibose (0.1 mg/kg)
   Fenofibrate-administered group (Group 4):
fenofibrate (50 mg/kg)
   Voglibose- and fenofibrate-administered group (Group 5): voglibose (0.1 mg/kg) + fenofibrate (50 mg/kg)
   Incidentally, as a control, a group in which neither STZ treatment nor test article administration were not conducted (Group 1), and a group in which after STZ treatment test article administration was not conducted (Group 2) were employed, and GLU concentrations before and after sucrose loading were also measured in the same manner with above.

As a result, Group 5 (voglibose- and fenofibrate-administered group) was significantly inhibited in the GLU concentration after sucrose loading compared with Group 3 (voglibose- administered group) and Group 4 (fenofibrate-administered group).

## Claims

1. A pharmaceutical composition including a combination of (a) at least one hyperlipidemic agent selected from the group consisting of a fibrate compound and a hydroxymethylglutaryl-CoA reductase inhibitor with (b) an α-glucosidase inhibitor, wherein the pharmaceutical is
(i) a pharmaceutical composition comprising the hyperlipidemic agent (a) and the α-glucosidase inhibitor (b), or
(ii) a pharmaceutical combination including a pharmaceutical component comprising the hyperlipidemic agent (a) and a pharmaceutical component comprising the α-glucosidase inhibitor (b).

2. A pharmaceutical composition according to claim 1, wherein the fibrate compound comprises at least one member selected from the group consisting of fenofibrate, bezafibrate, clinofibrate, clofibrate, simfibrate, fenofibric acid, and gemfibrozil, or a salt thereof.

3. A pharmaceutical composition according to claim 1, wherein the fibrate compound comprises at least one member selected from the group consisting of fenofibrate, and bezafibrate, or a salt thereof.

4. A pharmaceutical composition according to claim 1, wherein the hydroxymethylglutaryl-CoA reductase inhibitor comprises at least one statin compound selected from the group consisting of pravastatin, simvastatin, fluvastatin, atorvastatin, lovastatin, cerivastatin, pitavastatin, and rosvastatin, or a salt thereof.

5. A pharmaceutical composition according to claim 1, wherein the hydroxymethylglutaryl-CoA reductase inhibitor comprises at least one statin compound selected from the group consisting of pravastatin, and atorvastatin, or a salt thereof.

6. A pharmaceutical composition according to claim 1, wherein the α-glucosidase inhibitor (b) comprises at least one member selected from the group consisting of voglibose, acarbose, miglitol, and emiglitate, or a salt thereof.

7. A pharmaceutical composition according to claim 1, wherein the α-glucosidase inhibitor (b) comprises at least one member selected from the group consisting of voglibose and acarbose.

8. A pharmaceutical composition according to claim 1, wherein the proportion of the α-glucosidase inhibitor (b) is 0.001 to 50 parts by weight relative to 100 parts by weight of the hyperlipidemic agent (a).

9. A pharmaceutical composition according to claim 1, wherein the proportion of the α-glucosidase inhibitor (b) is 0.01 to 10 parts by weight relative to 100 parts by weight of the hyperlipidemic agent (a).

10. A pharmaceutical composition including a combination of fenofibrate and voglibose, which is
(i) a pharmaceutical composition comprising the fenofibrate and the voglibose, or
(ii) a pharmaceutical combination including a pharmaceutical component comprising the fenofibrate and a pharmaceutical component comprising the voglibose.

11. A pharmaceutical composition according to claim 1 or 10, which is an agent for the prophylaxis or treatment of metabolic syndrome.

12. A pharmaceutical composition according to claim 1 or 10, which is an agent for the prophylaxis or treatment of at least one symptom selected from the group consisting of hyperlipemia, diabetes, diabetes complications, a symptom of hyperglycemia after a meal in diabetics, impaired glucose tolerance (IGT), decrease of glucose tolerance, hypertension, hyperinsulinemia, hyperammonemia, obesity or a complication thereof, fatty liver, and hepatitis.

13. A pharmaceutical composition according to claim 1 or 10, which is an agent for the prophylaxis or treatment of hyperlipemia.

14. A pharmaceutical composition according to claim 1 or 10, which is an agent for the prophylaxis or treatment of at least one symptom selected from the group consisting of diabetes, diabetes complications and a symptom of hyperglycemia after a meal in diabetics.

15. A pharmaceutical composition according to claim 1, which is
(i) a pharmaceutical preparation comprising (a) a hyperlipidemic agent and (b) an α-glucosidase inhibitor, or
(ii) a pharmaceutical combination including a pharmaceutical preparation comprising the hyperlipidemic agent (a) and a pharmaceutical preparation comprising the α-glucosidase inhibitor (b).

16. Use of (a) at least one hyperlipidemic agent selected from the group consisting of a fibrate compound and a hydroxymethylglutaryl-CoA reductase inhibitor, and (b) an α-glucosidase inhibitor for preparing a pharmaceutical preparation.

17. A pharmaceutical composition reducing a side effect or dose of an α-glucosidase inhibitor, which includes a combination of (a) at least one hyperlipidemic agent selected from the group consisting of a fibrate compound and a hydroxymethylglutaryl-CoA reductase inhibitor and (b) an α-glucosidase inhibitor, wherein the pharmaceutical composition is
(i) a pharmaceutical composition comprising the hyperlipidemic agent (a) and the α-glucosidase inhibitor (b), or
(ii) a pharmaceutical combination including a pharmaceutical component comprising the hyperlipidemic agent (a) and a pharmaceutical component comprising the α-glucosidase inhibitor (b).

18. A method for preventing or treating at least one symptom selected from the group consisting of metabolic syndrome, hyperlipemia, diabetes, diabetes complications, a symptom of hyperglycemia after a meal in diabetics, impaired glucose tolerance (IGT), decrease of glucose tolerance,hypertension,hyperinsulinemia,hyperammonemia, obesity or a complication thereof, fatty liver, and hepatitis; wherein the method comprises
administering (a) at least one hyperlipidemic agent selected from the group consisting of a fibrate compound and a hydroxymethylglutaryl-CoA reductase inhibitor and (b) an α-glucosidase inhibitor to human or non-human animals to prevent or treat the symptom.
